# DEMANDE DE BREVET EUROPEEN

(11) **EP 1 609 458 A1**
(43) Date de publication de la demande: **28.12.2005**
(21) Numéro de dépôt: 05291274.8
(22) Date de dépôt: 14.06.2005
(51) Int. Cl.: A61K 7/13

(54) **Compositions tinctoriales comprenant un dérivé de 1,3-indanedione**

(30) Priorité: 14.06.2004 FR 0451167
(71) Demandeur: L'OREAL, 75008 Paris (FR)
(72) Inventeur: Plos, Grégory Gluck Heim Yoga, 158-0097 Tokyo (JP); Gourlaouen, Luc, 92600 Asnieres (FR)
(74) Mandataire: Fevrier, Murielle Françoise E.

(57) **Abrégé**

La présente invention concerne des compositions de coloration des matières kératiniques, en particulier des compositions de coloration capillaire contenant au moins un dérivé de 1,3-indanedione, un procédé de coloration utilisant de telles compositions et un agent de coloration multi-composants servant à la mise en oeuvre d'un tel procédé.

La présente invention permet en particulier d'obtenir une coloration des fibres kératiniques, tenace, résistante à la lumière et au lavage.

## Description

La présente invention concerne des compositions de coloration des matières kératiniques, en particulier des compositions de coloration capillaire contenant au moins un dérivé de 1,3-indanedione, un procédé de coloration utilisant de telles compositions et un agent de coloration multi-composants servant à la mise en oeuvre d'un tel procédé.

Depuis longtemps, de nombreuses personnes cherchent à modifier la couleur de leur peau, de leurs cils ou de leurs cheveux et en particulier à masquer leurs cheveux blancs. Pour ce faire, plusieurs technologies ont été développées.

Il est connu de teindre les fibres kératiniques humaines, et en particulier les cheveux, avec des compositions tinctoriales contenant des précurseurs de colorants d'oxydation, appelés généralement bases d'oxydation. Ces bases d'oxydation sont des composés incolores ou faiblement colorés qui, associés à des produits oxydants, donnent naissance par un processus de condensation oxydative à des composés colorés. Ces composés colorés sont insolubles et sont piégés à l'intérieur de la fibre capillaire.

On sait également que l'on peut faire varier les nuances obtenues avec ces bases d'oxydation en les associant à des coupleurs ou modificateurs de coloration. La variété des molécules mises en jeu au niveau des bases d'oxydation et des coupleurs permet l'obtention d'une riche palette de couleurs.

Les colorations obtenues présentent une bonne ténacité aux shampooings. Cependant, la réaction d'oxydation se fait à l'aide de produits oxydants tels l'eau oxygénée en milieu basique. Ces agents oxydants attaquent la kératine des cheveux dont les propriétés cosmétiques et mécaniques peuvent se dégrader fortement en cas de colorations répétées.

Il est aussi connu de teindre les fibres kératiniques humaines par une coloration directe consistant à appliquer sur les fibres kératiniques des colorants directs qui sont des molécules colorées et colorantes ayant une affinité pour les fibres. On peut citer à titre d'exemples de colorants directs utilisés classiquement les colorants nitrés, benzéniques, anthraquinoniques, nitropyridiniques, azoïques, azoïques cationiques, xanthéniques, acridiniques, aziniques ou de type triarylméthane ou des colorants naturels.

Il existe une autre méthode de coloration des matières kératiniques à partir d'indane-diones telles que les 1,2-indane diones et les 1,3-indane-diones. Ces méthodes de coloration sont par exemple décrites dans les demandes de brevet suivantes EP 1010419, EP 1013259, EP 1300134, WO 95/11001 et WO 99/18914.

Les colorations obtenues ainsi sont, certes, très chromatiques et n'entraînent pas une dégradation chimique de la kératine, mais présentent l'inconvénient de n'être que temporaires ou semi-permanentes, c'est-à-dire de s'estomper au mieux après seulement 4 à 5 shampooings.

Il subsiste par conséquent un besoin de systèmes et de procédés de coloration qui permettent l'obtention de bonnes ténacités sans impliquer l'utilisation d'agents oxydants susceptibles de dégrader les matières kératiniques.

Le but de la présente invention est de fournir de nouvelles compositions de teinture qui permettent de colorer les matières kératiniques, et en particulier les cheveux, avec des ténacités équivalentes voire supérieures à celles obtenues par coloration d'oxydation et ceci en l'absence d'agents oxydants forts préservant ainsi parfaitement les matières kératiniques.

Ce but est atteint avec la présente invention qui a pour objet une composition pour la teinture des matières kératiniques telles que la peau et les fibres kératiniques comprenant, dans un milieu approprié, au moins un dérivé de 1,3-indanedione choisi parmi les dérivés de formule (I), (II) ou (III) 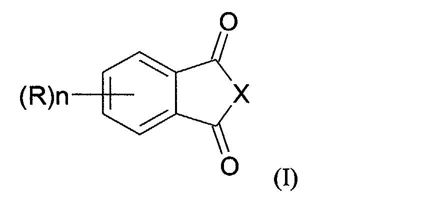 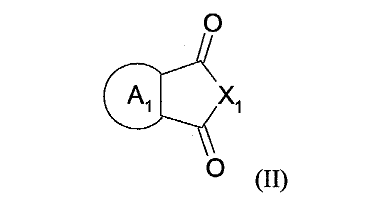 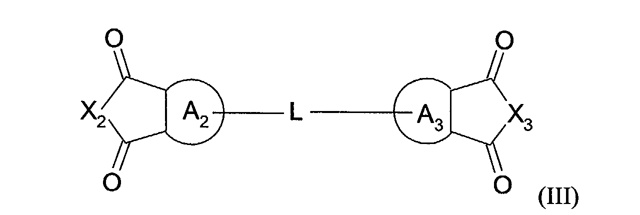 dans lesquelles
- X, X1, X2 et X3 représentent un radical CR¹R²,
- R1 et R2, indépendamment l'un de l'autre représentent un hydrogène, un halogène, un radical alkyle en C3-C 18, un radical alcényle en C2-C 18, un radical carboxylique, un radical hydroxycarbonylalkyle, un radical hydrogenocarbonyle, un radical hydrogénocarbonylalkyle, un radical alkylcarboxyalkyloxy, un radical cyano, un radical thiocyano, un radical nitro, un radical nitroso, un radical hydroxyalkyl, un radical alcoxy, un radical alcényloxy, un radical aryloxy, un radical amino, un radical alkylamino, un radical dialkylamino, un radical arylalkylamino, un radical diarylamino, un radical alcénylamino, un radical thio, un radical alkylthio, un radical arylthio, un radical alcénylthio, un radical aryle, un radical hétéroaryle, un radical ammonium quaternaire tel que tétraalkylammonium, pyridinium, benzothiazolium ou imidazolium,
- A1 représente un radical polyaromatique, condensé ou non, comprenant de 6 à 50 atomes de carbones, et pouvant contenir un ou plusieurs hétéroatomes choisis parmi l'azote, le soufre, l'oxygène et/ou le phosphore,
- A2 et A3, représentent indépendamment un radical mono ou polyaromatique, condensé ou non, comprenant de 6 à 50 atomes de carbones, et pouvant contenir un ou plusieurs hétéroatomes choisis parmi l'azote, le soufre, l'oxygène et/ou le phosphore,
- L représente une simple liaison ou un radical divalent aliphatique ou aromatique, le radical pouvant contenir un hétéroatome choisi parmi l'oxygène, le soufre, l'azote et le phosphore et pouvant être condensé avec A2 ou A3 ;
- n est un entier choisi entre 0 et 4 inclus,
- R représente un halogène, une groupement alkyle, un radical alcényle en C2-C 18, un radical carboxylique, un alcoxycarbonyle, un radical hydroxycarbonylalkyle, un radical hydrogenocarbonyle, un radical hydrogénocarbonylalkyle, un radical alkylcarboxyalkyloxy, un radical cyano, un radical thiocyano, un radical nitro, un radical nitroso, un radical sulfonato, un radical alkylsulfonamido, un radical hydroxy, un radical hydroxyalkyl, un radical alcoxy, un radical alcényloxy, un radical aryloxy, un radical amino, un radical alkylamino, un radical dialkylamino, un radical arylalkylamino, un radical diarylamino, un radical alcénylamino, un radical thio, un radical alkylthio, un radical arylthio, un radical alcénylthio, un radical silyle, un radical alkylsilyle, un radical alkylarylsilyle, un radical arylsilyle, un radical siloxy, un radical alkylsilyloxy, un radical arylsilyloxy, un radical aryle, un radical hétéroaryle, un radical ammonium quaternaire, un radical du type 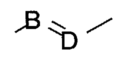 où B et D représentent indépendamment l'un de l'autre un atome de carbone ou d'azote et où W représente un cycle d'au moins 5 chaînons ou un polycycle, condensé ou non condensé, aromatique ou hétéroaromatique, l'hétéroatome pouvant être choisi parmi l'azote, l'oxygène, le soufre et/ou le phosphore,
avec les conditions suivantes
o lorsque X est CR1R2 et R1 et R2 représentent l'hydrogène, le composé de formule (I) est tel que 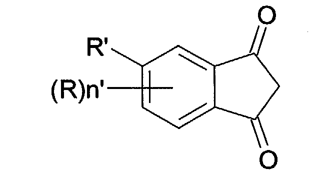 avec n' égal à 0, 1, 2 ou 3, et R' est tel que défini pour R à l'exception du radical méthoxy et chloro,
o lorsque X est CR1R2, n est égal à 0 et R1 est l'hydrogène alors R2 représente un atome d'halogène, ou un radical alkyle substitué comprenant de 1 à 4 atomes de carbone,
o lorsque X est CR1R2, n est égal à 0 et R1 est OH alors R2 est différent d'un radical amino, alkylamino, dialkylamino arylalkylamino, diarylamino, alcénylamino.

Les colorations ainsi obtenues présentent de bonnes chromaticités et se distinguent en particulier par une bonne ténacité aux agents extérieurs tels que la lumière, les shampooings, la sueur. De telles compositions sont en particulier utiles pour la teinture des fibres kératiniques, notamment les cheveux.

Dans la description ci-dessus, le nombre de carbone compris entre 1 et 18 correspond sauf indication contraire au nombre de carbones du radical alkyle. Lorsque le radical indiqué comprend un radical aryle, alors le nombre de carbone est de préférence compris entre 6 et 30. Selon un mode de réalisation particulier, le nombre de carbones des raducaux alkyle ou dérivés est compris entre 1 et 10, de préférence 1 à 6.

Dans le cadre de l'invention, on appelle radical polyaromatique, un radical qui contient au moins deux noyaux aromatiques, les noyaux du radical pouvant être condensés ou non condensés. Un radical monoaromatique est un radical qui contient un seul noyau aromatique.

Dans la formule (I) ci-dessus, R est de préférence choisi parmi un halogène, un radical alkyle, un radical aryle, un radical alcoxy, un radical aryloxy, un radical carboxylique, un radical alcoxycarbonyle, un radical nitro, amino, mono ou di alkylamino, un radical cyano, un radical thiocyano, un radical sulfonato, un radical alkylsulfonamido, ou n est égal à 0.

De préférence, R' représente un brome, un radical alkyle.

Selon un mode de réalisation particulier, R1 et R2 sont choisis parmi l'hydrogène, les halogènes en particulier le brome, un radical alcoxy, un radical alkyle.

Dans les dérivés de formule (II) ou (III), les cycles A1 sont par exemple choisis parmi les cycles naphtaléniques, anthracéniques, thiophènes, pyridines ou quinolines.

Les cycles A2 et A3, sont par exemple des groupements benzéniques, naphtaléniques, anthracéniques, thiophènes, pyridines ou quinolines. Selon un mode de réalisation particulier, A2 et A3 sont choisis parmi les cycles benzéniques, naphtaléniques, thiophènes ou pyridiniques.

Dans un mode de réalisation préféré de la présente invention, A1, A2 et A3 sont choisis de manière à former avec le noyau indane-dione un système à électrons π délocalisés.

Les cycles A1, A2 et A3 dans le cadre de l'invention peuvent être substitués ou non. A titre d'exemples de substituant de ces cycles, on peut citer les substituants donnés dans la définition de R.

A titre d'exemple de dérivés de formule (I), on peut citer

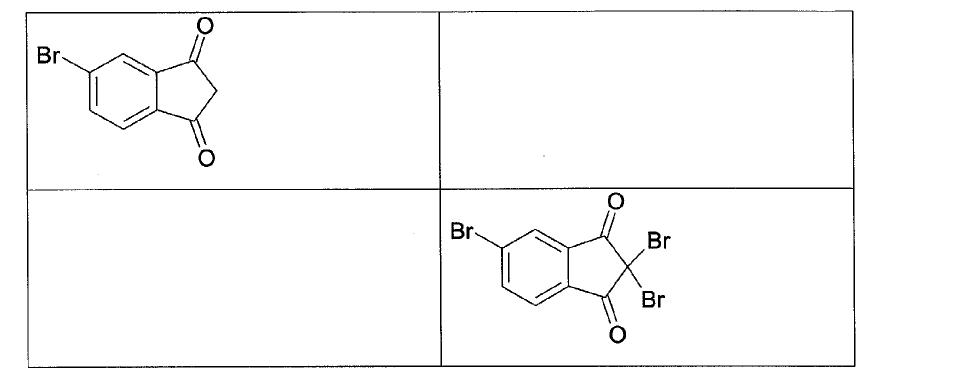

Selon un mode de réalisation particulier, le dérivé de formule (I) est choisi parmi les dérivés de formule : 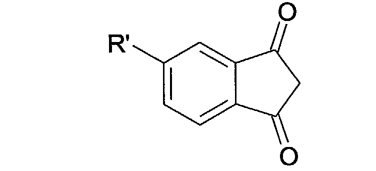 dans laquelle R' est choisi parmi un brome, un radical alkyle, un radical aryle, un radical alcoxy autre que méthoxy, un radical aryloxy, un radical carboxylique, un radical alcoxycarbonyle, un radical nitro, amino, mono ou di alkylamino, un radical cyano, un radical thiacyano, un radical sulfonato, un radical alkylsulfonamido.

A titre d'exemple de dérivés de formule (II), on peut citer les dérivés

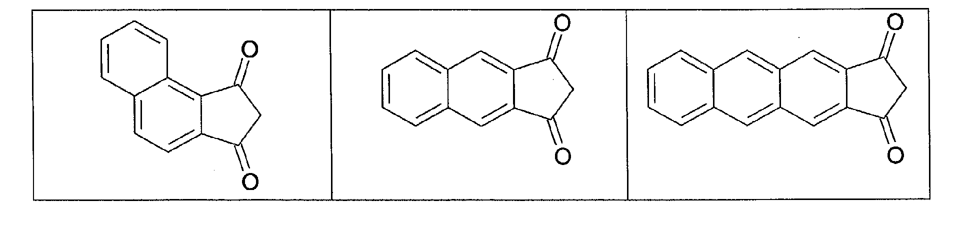

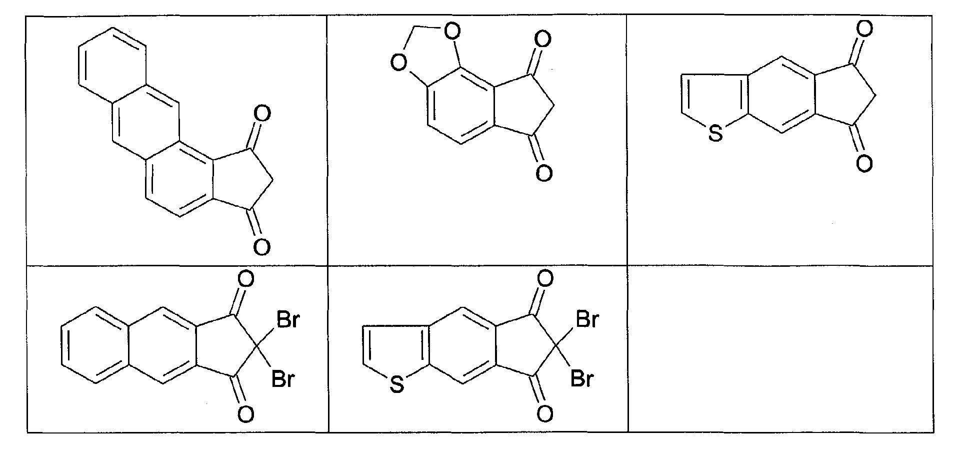

Selon un mode de réalisation particulier de l'invention, les dérivés de 1,3-indanedione de formule (II) sont choisis parmi les dérivés suivants : 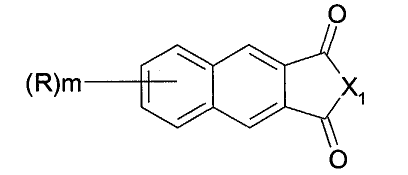 dans laquelle X1 représente CR1R2 tel que défini précédemment, R est tel que défini précédemment, m est compris entre 0 et 6, de préférence entre 0 et 4. Selon un mode de réalisation particulier, m est égal à 0 ou R représente un halogène, un radical alkyle, un radical alcoxy.

De préférence, R1 et R2 sont indépendamment choisis parmi un atome d'hydrogène ou un atome d'halogène, un radical alkyle, un radical alcoxy.

Selon un mode de réalisation particulier, les dérivés de formule (III) répondent à la formule

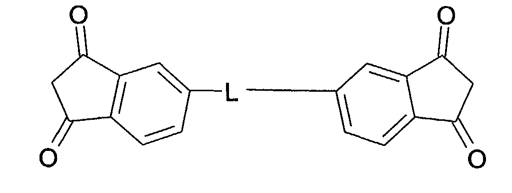

Selon l'invention, L représente de préférence un cycle d'au moins 6 chaînons ou un polycycle condensé ou non condensé, aromatique ou hétéroaromatique, ces cycles pouvant être substitués. Il est aussi possible pour L de former un cycle condensé sur A2 et/ou A3.

A titre de dérivés de formule (III), on peut citer

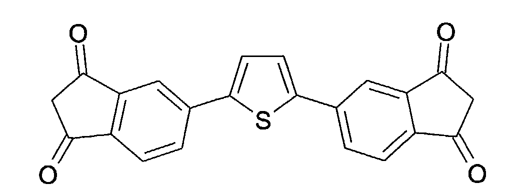

Dans le cadre de la présente invention, les groupements L, R1, R2, R, A1, A2 et A3 peuvent être non substitués ou substitués. Lorsqu'ils sont substitués, le ou les substituants peuvent être choisis parmi un ou plusieurs radicaux halogéno, alkyle en C1-C6, hydroxy, alcoxy en C1-C6, amino, imidazolyle, pyridinyle, mono- ou di-(alkyle en C1-C6)amino, mono- ou di-hydroxy(alkyl en C1-C6)amino, tri(alkyle en C1-C6)ammonio, groupements thio, (alkyle en C1-C6)thio, thio(alkyle en C1-C6), (alkyle en C1-C6)carbonyle, hydrogénocarbonyle, hydroxycarbonyle, (alcoxy en C1-C6)carbonyle, nitro, sulfonato ainsi que les groupes protonés correspondants tels que ammonio, imidazolio, et/ou pyridinio.

Les dérivés de formule (I), (II) ou (III) englobent, bien entendu, également les sels d'addition d'acides et les sels d'addition de bases correspondants.

Dans les radicaux définis ci dessus, les radicaux alkyles présentent de préférence de 1 à 10 atomes de carbone.

Les dérivés de 1,3-indanedione de formule (I), (II) ou (III) peuvent être obtenus à partir de méthodes de synthèse connues. En particulier, les synthèses peuvent être réalisées à partir de l'enseignement décrit dans les documents EP 1010419, EP 1013259, EP 1300134, WO 95/11001 et WO 99/18914 et des articles « Ninhydrin and ninhydrin analogs, Syntheses and applications", M.M. Joullié, T.R. Thompson, N.H. Nemeroff, *Tetrahedron,* Vol 47, n°42, 8791-8830, (1991) , The total synthesis of Ochrobirine, B. Nalliah, Q.A. Ahmed, R.H.F. Manske, R. Rodrigo, Canadian Journal of chemistry, 50, 1819 (1972), Synthesis of Ninhydrin analogs, R.R. Hark, PhD, Dissertation in chemistry, University of Pennsylvania, 1996.

Conformément à la présente invention, les dérivés de 1,3-indanedione de formule (I) décrits ci-dessus peuvent être utilisés seuls pour la coloration des matières kératiniques. En effet, ces composés sont capables de générer des molécules colorées avec les fonctions amine de la kératine (réaction colorée).

On peut aussi utiliser conjointement des composés de formule (I) avec au moins un activateur qui permet de modifier la cinétique de réaction du dérivé de 1,3-indanedione avec la matière kératinique. Un tel activateur peut être un agent oxydant, un agent réducteur, des acides de brönsted, un catalyseur métallique tels que les catalyseurs à base d'un métal de transition tel que le fer, le platine, le palladium, des protéines notamment des enzymes, des composés modifiant la force ionique tels que des sels tels que NaCl, des composés à hydrogène labile choisi parmi ceux comportant une fonction amine primaire ou secondaire et ceux comportant une fonction méthylène activé. On peut, bien entendu, utiliser également un mélange de tels composés.

Selon un mode de réalisation préféré, l'activateur chimique est un composé à hydrogène labile choisi parmi les composés comportant une fonction amine primaire ou secondaire et les composés comportant une fonction méthylène activé. De préférence, les composés à fonction amine primaire ou amine secondaire sont des amines aromatiques.

On peut citer à titre d'exemples de telles amines aromatiques la N,N-diméthyl-p-phénylènediamine, la N,N-diéthyl-p-phénylènediamine, la N-(2-hydroxy-éthyl)-N-éthyl-p-phénylènediamine, la N,N-bis-(2-hydroxyéthyl)-p-phénylène-diamine, la N-(2-méthoxyéthyl)-p-phénylènediamine, la 2,3-, 2,4- ou 2,5-dichloro-p-phénylène-diamine, la 2-chloro-p-phénylènediamine, le dibromhydrate de 2,5-dihydroxy-4-morpholinoaniline, le 2-, 3- ou 4-aminophénol, le 2-aminométhyl-4-aminophénol, le 2-hydroxyméthyl-4-aminophénol, l'ortho-phénylènediamine, la p-phénylènediamine, l'ortho-toluènediamine, le 2,5-diaminotoluène, le 2,5-diaminophénol, le 2,5-diaminophénéthol, le 4-amino-3-méthylphénol, le 2-(2,5-diaminophényl)-éthanol, le 2,4-diaminophénoxyéthanol, le 2-(2,5-diaminophénoxy)-éthanol, la 4-méthylaminoaniline, la 3-amino-4-(2'-hydroxyéthyloxy)aniline, la 3,4-méthylènediaminoaniline, la 3,4-méthylènedioxyaniline, le 3-amino-2,4-dichlorophénol, le 4-méthylaminophénol, le 2-méthyl-5-aminophénol, le 3-méthyl-4-aminophénol, le 2-méthyl-5-(2-hydroxyéthylamino)phénol, le 6-méthyl-3-amino-2-chlorophénol, le 2-méthyl-5-amino-4-chlorophénol, le 3,4-méthylènedioxyphénol, le 5-(2-hydroxyéthylamino)-4-méthoxy-2-méthylphénol, le 4-amino-2-hydroxyméthylphénol, le 1,3-diamino-2,4-diméthoxybenzène, l'acide 2-, 3-, 4-aminobenzoïque, l'acide 2-amino-, 3-amino ou 4-aminophénylacétique, l'acide 2,3-, 2,4-, 2,5-, 3,4- ou 3,5-diaminobenzoïque, l'acide 4-amino- ou 5-amino-salicylique, l'acide 3-amino-4-hydroxybenzoïque, l'acide 4-amino-3-hydroxybenzoïque, l'acide 2-amino-, 3-amino ou 4-aminobenzènesulfonique, l'acide 3-amino-4-hydroxybenzènesulfonique, l'acide 4-amino-3-hydroxynaphtalène-1-sulfonique, l'acide 6-amino-7-hydroxynaphtalène-2-sulfonique, l'acide 7-amino-4-hydroxynaphtalène-2-sulfonique, l'acide 4-amino-5-hydroxynaphtalène-2,7-disulfonique, l'acide 3-amino-2-naphtoïque, l'acide 3-aminophtalique, l'acide 5-aminoisophtalique, le 1,3,5-triaminobenzène, le 1,2,4-triaminobenzène, le 1,2,4,5-tétraaminobenzène, le 2,4,5-triaminophénol, le pentaaminobenzène, l'hexaaminobenzène, le 2,4,6-triaminorésorcinol, le 4,5-diaminopyrochatécol, le 4,6-diaminopyrogallol, le 3,5-diamino-4-hydroxypyrochatécol, et les anilines aromatiques et phénols aromatiques comportant un autre résidu aromatique, correspondant à la formule (Ia) 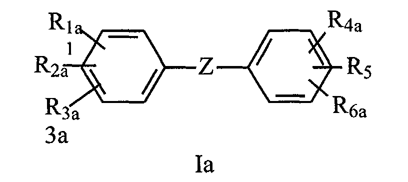 dans laquelle
R^{1a} représente un groupe hydroxy ou amino éventuellement substitués par un groupe alkyle en C₁₋₄, hydroxyalkyle en C₁₋₄ ou (alcoxy en C₁₋₄)-(alkyle en C₁₋₄),
R^{2a}, R^{3a}, R^{4a}, R^{5a} et R^{6a} représentent chacun indépendamment un atome d'hydrogène, un groupe hydroxy ou un groupe amino, éventuellement substitués par un groupe alkyle en C₁₋₄, hydroxyalkyle en C₁₋₄ ou (alcoxy en C₁₋₄)-(alkyle en C₁₋₄), ou un groupe acide carboxylique ou sulfonique,
Z représente une liaison directe, une chaîne hydrocarbonée en C₁₋₄, saturée ou insaturée, éventuellement hydroxylée, un groupe carbonyle, sulfonyle ou imino, un atome d'oxygène ou de soufre, ou un groupe de formule Q-(CH₂-P-CH₂-Q')ₒ où P représente une liaison directe ou un groupe -CH₂- ou -CHOH-, Q et Q' représentent, indépendamment l'un de l'autre, un atome d'oxygène, un groupe NR⁷ où R⁷ représente un atome d'hydrogène, un groupe alkyle en C₁₋₄ ou hydroxyalkyle en C₁₋₄, ou un groupe O-(CH₂)ₚNH ou NH-(CH2)ₚ'-O où p et p' valent 2 ou 3 et o est un nombre entre 1 et 4.

Les amines primaires ou secondaires non-aromatiques sont par exemple le 2-aminoéthanol, la 2-méthoxyéthylamine, la 2-éthoxyéthylamine, le 2-(2-aminoéthoxy)-éthanol, le 2- ou 3-aminopropanol, la 2,3-dihydroxypropylamine, la 4-hydroxypropylamine, le 2-aminopropane-1,3-diol, le 2-amino-2-méthylpropanol, le 2-amino-2-méthylpropane-1,3-diol, le 2-amino-2-hydroxyméthylpropane-1,3-diol, la tétrahydropentylamine, les pentahydroxyhexylamines telles que la glucamine, la D-glucosamine, la D-galactosamine, le 1,2-diaminoéthane, le 1,2- ou 1,3-diaminopropane, le 1,3-diamino-2-propanol, la 2-(2-amnoéthylamino)-éthylamine, le 2-(2-aminoéthylamino)éthanol, la 3-(2-aminoéthylamino)-propylamine et le 3-(2-aminoéthylamino)propanol.

Les composés à fonction méthylène activé sont choisis par exemple parmi les composés suivants : l'iodure de 1,2,3,3-tétraméthyl-3H-indolium, le p-toluènesulfonate de 1,2,3,3-tétraméthyl-3H-indolium, le méthanesulfonate de 1,2,3,3-tétraméthyl-3H-indolium, la 1,3,3-triméthyl-2-méthylène-indoline, l'iodure de 2,3-diméthylbenzothiazolium, le p-toluènesulfonate de 2,3-diméthylbenzothiazolium, la rhodanine, l'acide rhodanine-3-acétique, l'iodure de 1-éthyl-2-quinaldinium, l'iodure de 1-méthyl-2-quinaldinium, l'acide barbiturique, l'acide thiobarbiturique, l'acide 1,3-diméthylthiobarbiturique, l'acide diéthylthiobarbiturique, l'oxindole, l'acétate de 3-indoxyle, la coumarone et la 1-méthyl-3-phényl-2-pyrazolinone.

Ces amines primaires et secondaires et ces composés à fonctions méthylène activé ainsi que d'autres composés à hydrogène labile sont décrits également dans les demandes de brevet DE 43 17 855, DE 197 17 222, DE 198 45 481 et DE 197 45 355.

Lorsque les dérivés de 1,3-indanedione de formule (I), (II) ou (III) sont utilisés en combinaison avec une amine primaire ou secondaire ou avec un composé à fonction méthylène activé, il est nécessaire de conserver ces différents réactifs séparément pour éviter une réaction colorée prématurée. La mise en contact des réactifs ne se fait alors qu'immédiatement avant application sur les cheveux, par mélange extemporané de deux compositions contenant respectivement les dérivés de 1,3-indanedione et les composés à hydrogène labile. La mise en contact des réactifs peut se faire aussi directement sur le cheveu par application successive des différents réactifs.

L'invention a par conséquent également pour objet un agent de coloration multi-composants comportant
- en tant que premier composant, une composition (a) contenant au moins un dérivé de 1,3-indanedione de formule (I), (II) ou (III) ou un mélange de ces dérivés, et
- en tant que deuxième composant, une composition (b) contenant au moins un activateur qui permet de modifier la cinétique de réaction du dérivé de 1,3-indanedione.

Selon un mode de réalisation particulier, l'activateur est un composé à fonction amine primaire ou secondaire ou au moins un composé à fonction méthylène activé, tels que décrits ci-dessus.

Cet agent de coloration multi-composants se présente de préférence sous forme d'un kit multi-compartiments, avec au moins un premier compartiment contenant la composition (a) et au moins un deuxième compartiment contenant la composition (b).

Selon la présente invention, la composition tinctoriale contient en plus du ou des dérivés 1,3-indanedione de formule (I), (II) ou (III) au moins un principe actif cosmétique.

Les principes actifs cosmétiques présents dans les compositions de la présente invention sont choisis par exemple parmi les vitamines, les saccharides, les oligosaccharides, les polysaccharides hydrolysés ou non, modifiés ou non, les acides aminés, les oligopeptides, les peptides, les protéines hydrolysées ou non, modifiées ou non, les polyacides aminés, les enzymes, les acides et alcools gras ramifiés ou non, les cires animales, végétales ou minérales, les céramides et les pseudo-céramides, les acides organiques hydroxylés, les filtres UV, les agents anti-oxydants et les agents anti-radicaux libres, les agents chélatants, les agents antipelliculaires, les agents régulateurs de séborrhée, les agents apaisants, les agents tensioactifs cationiques, anioniques, non-ioniques ou amphotères, les polymères cationiques, anioniques, neutres ou amphotères, les silicones organomodifiées ou non, les huiles minérales, végétales ou animales, les polyisobutènes et poly(α-oléfines), les esters gras, les polymères anioniques sous forme dissoute ou dispersée, les polymères non-ioniques sous forme dissoute ou dispersés, les agents réducteurs, les solvants, les colorants capillaires tels que les colorants directs ou les précurseurs de coloration d'oxydation (bases et/ou coupleurs) différents des composés à fonction amine primaire ou secondaire revendiqués, les agents oxydants tels que le peroxyde d'hydrogène éventuellement associé à des persels, les pigments et leurs mélanges.

Le principe actif cosmétique est présent de préférence à raison de 0,001 à 50 % en poids, en particulier de 0,01 à 20 % en poids, et idéalement à raison de 0,1 à 10 % en poids, rapporté au poids total de la composition cosmétique.

Dans un mode de réalisation préféré de la composition cosmétique tinctoriale selon l'invention, le principe actif cosmétique est un agent tensioactif et/ou un agent polymérique (polymère), ces agents pouvant être de nature non-ionique, cationique, anionique ou amphotère.

Les compositions de la présente invention ont de préférence un pH compris entre 2 et 12, et en particulier entre 6 et 11.

La teneur en dérivé de 1,3-indanedione de formule (I), (II) ou (III) est de préférence comprise entre 0,0001 % et 30 % en poids par rapport au poids total de la composition.

Les composés à hydrogène labile lorsqu'ils sont présents dans la composition de l'invention sont de préférence présents à raison de 0,0001 à 30% en poids par rapport au poids total de la composition.

La présente invention a en outre pour objet un procédé de coloration comprenant l'application, sur une matière kératinique, en particulier les fibres kératiniques, de la composition de l'invention telle que décrite ci-dessus. Cette composition est laissée en contact avec la matière kératinique pendant un temps suffisant pour l'obtention de la coloration désirée. Ce temps de pose est généralement compris entre 5 minutes et 1 heure, et en particulier entre 15 et 30 minutes. La réaction colorée entre les dérivés de 1,3-indanedione et les fonctions amine de la kératine ou les composés à hydrogène labile éventuellement présents, peut être accélérée par chauffage après application sur la matière kératinique. La température de chauffage ne dépasse de préférence pas 250 °C, et est en particulier inférieure ou égale à 60 °C.

Après obtention de la coloration souhaitée, la matière kératinique est de préférence rincée et lavée.

Lorsqu'on utilise des composés à hydrogène labile tels que des amines primaires ou secondaires ou des composés à fonction méthylène activé, l'application des réactifs participant à la réaction colorée peut également se faire en deux temps, autrement dit, on peut appliquer successivement deux compositions différentes, une composition (a) contenant au moins un dérivé de 1,3-indanedione de formule (I) et une composition (b) contenant au moins un composé comportant une fonction amine primaire ou secondaire ou une fonction méthylène activé.

La présente invention a par conséquent également pour objet un procédé de coloration en deux temps comprenant le fait d'appliquer sur les cheveux l'une après l'autre, dans n'importe quel ordre, une composition (a) et une composition (b) telles que définies ci-dessus pour l'agent de coloration multi-composants.

Cette application séparée des deux compositions réactives présente l'avantage d'éviter la manipulation de compositions colorées et diminue ainsi les risques de souillure de matériaux tels que les vêtements.

Selon un mode de réalisation particulier, le procédé de coloration est un procédé de coloration des fibres kératiniques, en particuliers les cheveux.

Il est aussi possible d'insérer une étape de rinçage intermédiaire entre l'application de la composition (a) et l'application de la composition (b).

De manière analogue à celle décrite ci-dessus, il est possible de chauffer la matière kératinique sur laquelle est appliquée la ou les composition (a) et (b).

Selon un mode de réalisation particulier, les procédés de coloration décrits ci dessus sont des procédés de coloration des fibres kératiniques, en particulier les cheveux.

L'invention a enfin pour objet l'utilisation des compositions décrites ci dessus pour la teinture des matières kératiniques, en particulier les cheveux.

La composition de teinture suivante a été réalisée :

| | |
|---|---|
| Dérivé de 1,3-indanedione (a1) | 10-2 mole% |
| Ethanol | 50% |
| NaOH | q.s. pH 7 |
| Eau distillée | q.s.p. 100% |

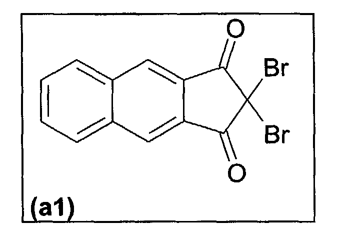

La composition est appliquée sur une mèche de cheveux naturels gris à 90 % de blancs et sur une mèche de cheveux permanentés gris à 90 % de blancs pendant 30 minutes à la température de 60°C. A l'issue de la coloration, les mèches sont rincées et séchées.

Les résultats colorimétriques sont donnés dans le tableau ci-dessous :

| | **Evaluation visuelle** |
|---|---|
| Cheveux naturels | cuivré |
| Cheveux permanentés | cuivré |

## Revendications

1. Composition pour la teinture des matières kératiniques comprenant, dans un milieu approprié, au moins un dérivé de 1,3-indanedione choisi parmi les dérivés de formule (I), (II) ou (III) 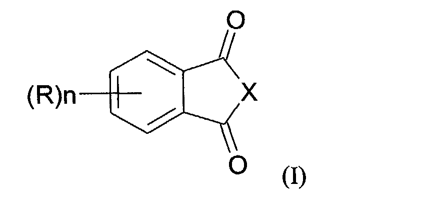 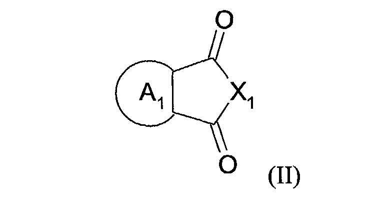 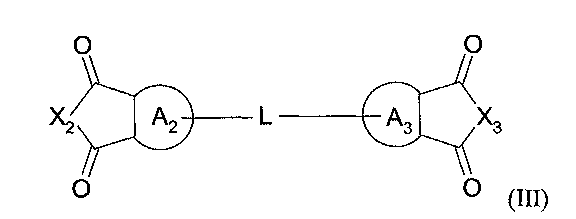 dans lesquelles
• X, X1, X2 et X3 représentent un radical CR¹R²,
• R1 et R2, indépendamment l'un de l'autre représentent un hydrogène, un halogène, un radical alkyle en C3-C18, un radical alcényle en C2-C 18, un radical carboxylique, un radical hydroxycarbonylalkyle, un radical hydrogenocarbonyle, un radical hydrogénocarbonylalkyle, un radical alkylcarboxyalkyloxy, un radical cyano, un radical thiocyano, un radical nitro, un radical nitroso, un radical hydroxyalkyl, un radical alcoxy, un radical alcényloxy, un radical aryloxy, un radical amino, un radical alkylamino, un radical dialkylamino, un radical arylalkylamino, un radical diarylamino, un radical alcénylamino, un radical thio, un radical alkylthio, un radical arylthio, un radical alcénylthio, un radical aryle, un radical hétéroaryle, un radical ammonium quaternaire,
• A1 représente un radical polyaromatique, condensé ou non, comprenant de 6 à 50 atomes de carbones, et pouvant contenir un ou plusieurs hétéroatomes choisis parmi l'azote, le soufre, l'oxygène et/ou le phosphore,
• A2 et A3, représentent indépendamment un radical mono ou polyaromatique, condensé ou non, comprenant de 6 à 50 atomes de carbones, et pouvant contenir un ou plusieurs hétéroatomes choisis parmi l'azote, le soufre, l'oxygène et/ou le phosphore,
• L représente une simple liaison ou un radical divalent aliphatique ou aromatique, le radical pouvant contenir un hétéroatome choisi parmi l'oxygène, le soufre, l'azote et le phosphore et pouvant être condensé avec A2 ou A3 ;
• n est un entier choisi entre 0 et 4 inclus,
• R représente un halogène, une groupement alkyle, un radical alcényle, un radical carboxylique, un alcoxycarbonyle, un radical hydroxycarbonylalkyle , un radical hydrogenocarbonyle, un radical hydrogénocarbonylalkyle, un radical alkylcarboxyalkyloxy, un radical cyano, un radical thiocyano, un radical nitro, un radical nitroso, un radical sulfonato, un radical alkylsulfonamido, un radical hydroxy, un radical hydroxyalkyl, un radical alcoxy, un radical alcényloxy, un radical aryloxy, un radical amino, un radical alkylamino, un radical dialkylamino, un radical arylalkylamino, un radical diarylamino, un radical alcénylamino, un radical thio, un radical alkylthio, un radical arylthio, un radical alcénylthio, un radical silyle, un radical alkylsilyle en C1-C18, un radical alkylarylsilyle, un radical arylsilyle, un radical siloxy, un radical alkylsilyloxy, un radical arylsilyloxy, un radical aryle, un radical hétéroaryle, un radical ammonium quaternaire, un radical du type 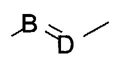 où B et D représentent indépendamment l'un de l'autre un atome de carbone ou d'azote et où W représente un cycle d'au moins 5 chaînons ou un polycycle, condensé ou non condensé, aromatique ou hétéroaromatique, l'hétéroatome pouvant être choisi parmi l'azote, l'oxygène, le soufre et/ou le phosphore,
**avec les conditions suivantes**
o lorsque X est CR1R2 et R1 et R2 représentent l'hydrogène, le composé de formule (I) est tel que 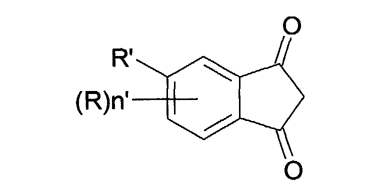 avec n' égal à 0, 1, 2 ou 3, et R' est tel que défini pour R à l'exception des radicaux methoxy et chloro,
o lorsque X est CR1R2, n est égal à 0 et R1 est l'hydrogène alors R2 représente un atome d'halogène, ou un radical alkyle substitué comprenant de 1 à 4 atomes de carbone,
o lorsque X est CR1R2, n est égal à 0 et R1 est OH alors R2 est différent d'un radical amino, alkylamino, dialkylamino arylalkylamino, diarylamino, alcénylamino.

2. Composition selon la revendication 1 dans laquelle R est choisi parmi un halogène, un radical alkyle, un radical aryle, un radical alcoxy, un radical aryloxy, un radical carboxylique, un radical alcoxycarbonyle, un radical nitro, amino, mono ou di alkylamino, un radical cyano, un radical thiocyano, un radical sulfonato, un radical alkylsulfonamido, ou n est égal à 0.

3. Composition selon la revendication 2 dans laquelle R' représente un brome, un radical alkyle..

4. Composition selon l'une quelconque des revendications 1 à 3 dans laquelle R1 et R2 sont choisis parmi l'hydrogène, les halogènes en particulier le brome, un radical alcoxy, un radical alkyle.

5. Composition selon l'une quelconque des revendications précédentes dans laquelle les dérivés de formule (II) sont tels que les cycles A1 sont choisis parmi les cycles naphtaléniques, anthracéniques, thiophènes, pyridines ou quinolines.

6. Composition selon l'une quelconque des revendications 1 à 4 dans laquelle les dérivés de formule (III) sont tels que les cycles A2 et A3, sont des groupements benzéniques, naphtaléniques, anthracéniques, thiophènes, pyridines ou quinolines.

7. Composition selon la revendication 6 dans laquelle les dérivés de formule (III) sont tels que A2 et A3 sont choisis parmi les cycles benzéniques, naphtaléniques, thiophènes ou pyridiniques.

8. Composition selon l'une quelconque des revendications précédentes dans laquelle A1, A2 et A3 sont choisis de manière à former avec le noyau indanedione un système à électrons π délocalisés.

9. Composition selon l'une quelconque des revendications 1 à 8 dans laquelle les dérivés de formule (I) sont :

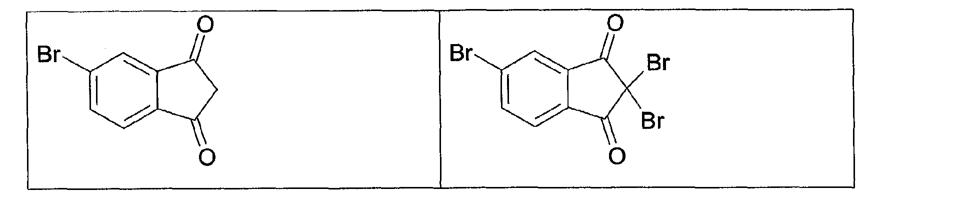

10. Composition selon la revendication 1 dans laquelle le dérivé de formule (I) est choisi parmi les dérivés de formule : 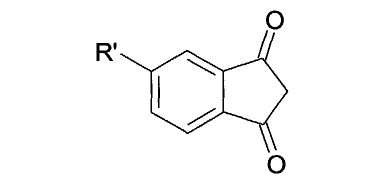 dans laquelle R' est choisi parmi un brome, un radical alkyle, un radical aryle, un radical alcoxy autre que méthoxy, un radical aryloxy, un radical carboxylique, un radical alcoxycarbonyle, un radical nitro, amino, mono ou di alkylamino, un radical cyano, un radical thiacyano, un radical sulfonato, un radical alkylsulfonamido.

11. Composition selon la revendication 1 dans laquelle les dérivés de formule (II) sont :

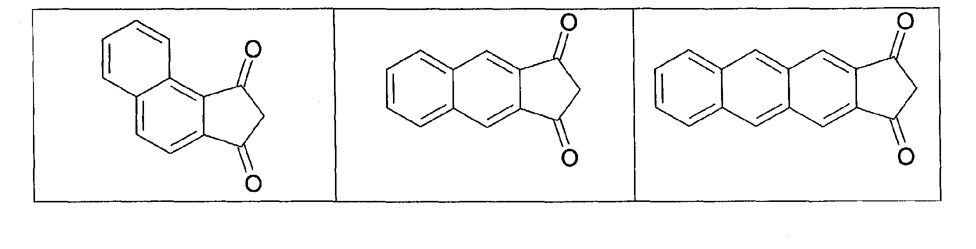

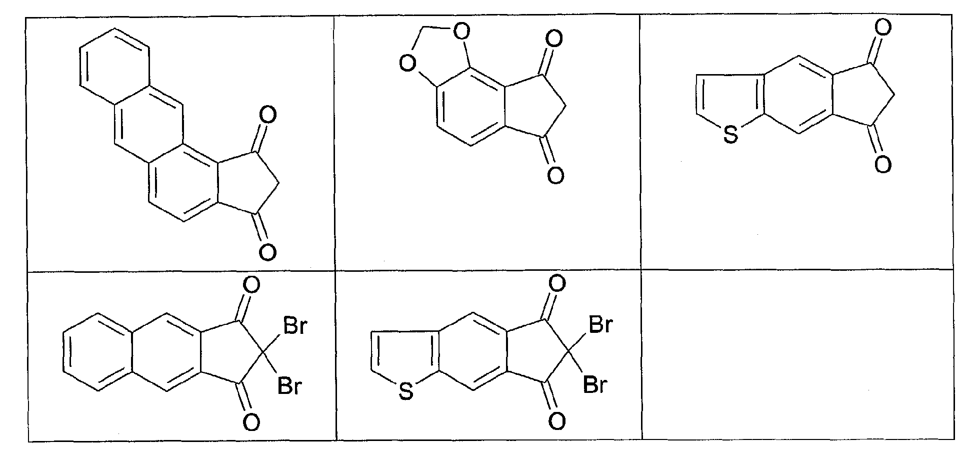

12. Composition selon la revendication 1 dans laquelle les dérivés de 1,3-indanedione de formule (II) sont choisis parmi les dérivés suivants : 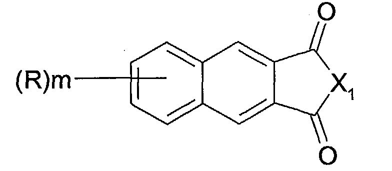 dans laquelle X1 représente CR1R2 , R1, R2 et R étant tel que définis précédemment et m est compris entre 0 et 6.

13. Composition selon la revendication 12 dans laquelle m est égal à 0 ou R représente un halogène, un radical alkyle, un radical alcoxy.

14. Composition selon la revendication 12 dans laquelle R1 et R2 sont indépendamment choisis parmi un atome d'hydrogène ou un atome d'halogène, un radical alkyle, un radical alcoxy.

15. Composition selon la revendication 1 dans laquelle les dérivés de formule (III) répondent à la formule 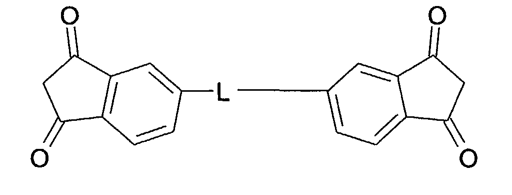 dans laquelle L représente un cycle d'au moins 6 chaînons ou un polycycle condensé ou non condensé, aromatique ou hétéroaromatique, ces cycles pouvant être substitués.

16. Composition selon la revendication 15 dans laquelle L forme un cycle condensé sur A2 et/ou A3.

17. Composition selon la revendication 15 dans laquelle les dérivés de formule (III) sont :

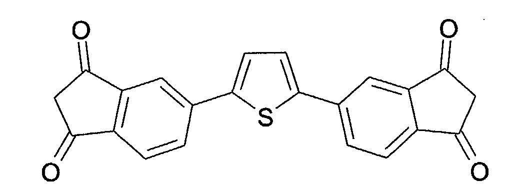

18. Composition selon l'une quelconque des revendications précédentes dans laquelle les groupements L, R1, R2, R, A1, A2 et A3 sont non substitués ou substitués par un ou plusieurs groupements halogéno, alkyle en C₁-C₆, hydroxy, alcoxy en C₁-C₆, amino, imidazolyle, pyridinyle, mono- ou di-(alkyle en C₁-C₆)amino, mono- ou di-hydroxy(alkyl en C₁-C₆)amino, tri(alkyle en C₁-C₆)ammonio, thio, (alkyle en C₁-C₆)thio, thio(alkyle en C₁-C₆), (alkyle en C₁-C₆)carbonyle, hydrogénocarbonyle, hydroxycarbonyle, (alcoxy en C₁-C₆)carbonyle, nitro, sulfonato, les groupes protonés.

19. Composition selon l'une quelconque des revendications précédentes comprenant de plus au moins un composé comportant une fonction amine primaire ou secondaire ou au moins un composé à fonction méthylène activé ou un mélange de ceux-ci.

20. Composition selon la revendication 19 dans laquelle le composé comportant une fonction amine primaire ou secondaire est une amine aromatique choisie parmi la N,N-diméthyl-p-phénylènediamine, la N,N-diéthyl-p-phénylènediamine, la N-(2-hydroxy-éthyl)-N-éthyl-p-phénylènediamine, la N,N-bis-(2-hydroxyéthyl)-p-phénylène-diamine, la N-(2-méthoxyéthyl)-p-phénylènediamine, la 2,3-, 2,4- ou 2,5-dichloro-p-phénylène-diamine, la 2-chloro-p-phénylènediamine, le dibromhydrate de 2,5-dihydroxy-4-morpholinoaniline, le 2-, 3- ou 4-aminophénol, le 2-aminométhyl-4-aminophénol, le 2-hydroxyméthyl-4-aminophénol, l'ortho-phénylènediamine, la p-phénylènediamine, l'ortho-toluènediamine, le 2,5-diaminotoluène, le 2,5-diaminophénol, le 2,5-diaminophénéthol, le 4-amino-3-méthylphénol, le 2-(2,5-diaminophényl)-éthanol, le 2,4-diaminophénoxyéthanol, le 2-(2,5-diaminophénoxy)-éthanol, la 4-méthylaminoaniline, la 3-amino-4-(2'-hydroxyéthyloxy)aniline, la 3,4-méthylènediaminoaniline, la 3,4-méthylènedioxyaniline, le 3-amino-2,4-dichlorophénol, le 4-méthylaminophénol, le 2-méthyl-5-aminophénol, le 3-méthyl-4-aminophénol, le 2-méthyl-5-(2-hydroxyéthylamino)phénol, le 6-méthyl-3-amino-2-chlorophénol, le 2-méthyl-5-amino-4-chlorophénol, le 3,4-méthylènedioxyphénol, le 5-(2-hydroxyéthylamino)-4-méthoxy-2-méthylphénol, le 4-amino-2-hydroxyméthylphénol, le 1,3-diamino-2,4-diméthoxybenzène, l'acide 2-, 3-, 4-aminobenzoïque, l'acide 2-amino-, 3-amino ou 4-aminophénylacétique, l'acide 2,3-, 2,4-, 2,5-, 3,4- ou 3,5-diaminobenzoïque, l'acide 4-amino- ou 5-amino-salicylique, l'acide 3-amino-4-hydroxybenzoïque, l'acide 4-amino-3-hydroxybenzoïque, l'acide 2-amino-, 3-amino ou 4-aminobenzènesulfonique, l'acide 3-amino-4-hydroxybenzènesulfonique, l'acide 4-amino-3-hydroxynaphtalène-1-sulfonique, l'acide 6-amino-7-hydroxynaphtalène-2-sulfonique, l'acide 7-amino-4-hydroxynaphtalène-2-sulfonique, l'acide 4-amino-5-hydroxynaphtalène-2,7-disulfonique, l'acide 3-amino-2-naphtoïque, l'acide 3-aminophtalique, l'acide 5-aminoisophtalique, le 1,3,5-triaminobenzène, le 1,2,4-triaminobenzène, le 1,2,4,5-tétraaminobenzène, le 2,4,5-triaminophénol, le pentaaminobenzène, l'hexaaminobenzène, le 2,4,6-triaminorésorcinol, le 4,5-diaminopyrochatécol, le 4,6-diaminopyrogallol, le 3,5-diamino-4-hydroxypyrochatécol, et les anilines aromatiques et phénols aromatiques comportant un autre résidu aromatique, correspondant à la formule (A) suivante 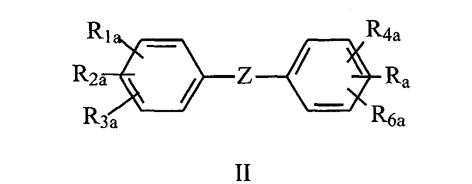 dans laquelle
R₁a représente un groupe hydroxy ou amino éventuellement substitués par un groupe alkyle en C₁₋₄, hydroxyalkyle en C₁₋₄ ou (alcoxy en C₁₋₄)-(alkyle en C₁₋₄),
R₂a, R₃a, R₄a, R₅a et R₆a représentent chacun indépendamment un atome d'hydrogène, un groupe hydroxy ou un groupe amino, éventuellement substitués par un groupe alkyle en C₁₋₄, hydroxyalkyle en C₁₋₄ ou (alcoxy en C₁₋₄)-(alkyle en C₁₋₄), ou un groupe acide carboxylique ou sulfonique,
Z représente une liaison directe, une chaîne hydrocarbonée en C₁₋₄, saturée ou insaturée, éventuellement hydroxylée, un groupe carbonyle, sulfonyle ou imino, un atome d'oxygène ou de soufre, ou un groupe de formule Q-(CH₂-P-CH₂-Q')ₒ où P représente une liaison directe ou un groupe -CH₂- ou -CHOH-, Q et Q' représentent, indépendamment l'un de l'autre, un atome d'oxygène, un groupe NR₇a où R₇ représente un atome d'hydrogène, un groupe alkyle en C₁₋₄ ou hydroxyalkyle en C₁₋₄, ou un groupe O-(CH₂)ₚNH ou NH-(CH₂)_{p'}-O où p et p' valent 2 ou 3 et o est un nombre entre 1 et 4, ou une amine aliphatique choisie parmi le 2-aminoéthanol, la 2-méthoxyéthylamine, la 2-éthoxyéthylamine, le 2-(2-aminoéthoxy)-éthanol, le 2- ou 3-aminopropanol, la 2,3-dihydroxypropylamine, la 4-hydroxypropylamine, le 2-aminopropane-1,3-diol, le 2-amino-2-méthylpropanol, le 2-amino-2-méthylpropane-1,3-diol, le 2-amino-2-hydroxyméthylpropane-1,3-diol, la tétrahydropentylamine, les pentahydroxyhexylamines telles que la glucamine, la D-glucosamine, la D-galactosamine, le 1,2-diaminoéthane, le 1,2- ou 1,3-diaminopropane, le 1,3-diamino-2-propanol, la 2-(2-amnoéthylamino)-éthylamine, le 2-(2-aminoéthylamino)éthanol, la 3-(2-aminoéthylamino)-propylamine et le 3-(2-aminoéthylamino)propanol.

21. Composition selon la revendication 20 dans laquelle le composé à fonction méthylène activé est choisi parmi l'iodure de 1,2,3,3-tétraméthyl-3H-indolium, le p-toluènesulfonate de 1,2,3,3-tétraméthyl-3H-indolium, le méthanesulfonate de 1,2,3,3-tétraméthyl-3H-indolium, la 1,3,3-triméthyl-2-méthylène-indoline, l'iodure de 2,3-diméthylbenzothiazolium, le p-toluènesulfonate de 2,3-diméthyl-benzothiazolium, la rhodanine, l'acide rhodanine-acétique, l'iodure de 1-éthyl-2-quinaldinium, l'iodure de 1-méthyl-2-quinaldinium, l'acide barbiturique, l'acide thiobarbiturique, l'acide 1,3-diméthylthiobarbiturique, l'acide diéthylthiobarbitique, l'oxindole, l'acétate de 3-indoxyle, la coumarine et la 1-méthyl-3-phényl-2-pyrazinone.

22. Composition selon l'une quelconque des revendications précédentes, dans laquelle la composition a un pH compris entre 2 et 12, de préférence entre 6 et 11.

23. Composition selon l'une quelconque des revendications précédentes dans laquelle la concentration du dérivé de formule (I), (II) ou (III) est comprise entre 0,0001 % et 30 %, rapporté au poids total de la composition.

24. Composition selon l'une quelconque des revendications précédentes, dans laquelle la concentration du composé à fonction méthylène activé ou du composé comportant une fonction amine primaire ou secondaire est comprise entre 0,0001 et 30%, rapporté au poids total de la composition.

25. Composition selon l'une quelconque des revendications précédentes comprenant au moins un agent tensioactif et/ou un agent polymérique de nature non ionique, cationique, anionique ou amphotère.

26. Agent de coloration multi-composant comportant
• en tant que premier composant, une composition (a) contenant au moins un dérivé de formule (I), (II) ou (III) tel que défini dans les revendications 1 à 23, ou un mélange de ces composés, et
• en tant que deuxième composant, une composition (b) contenant au moins un activateur qui permet de modifier la cinétique de réaction du dérivé de 1,3-indanedione.

27. Agent selon la revendication 26 dans lequel l'activateur est un composé comportant une fonction amine primaire ou secondaire ou au moins un composé à fonction méthylène activé tels que définis dans les revendications 24 ou 25 ou un mélange de ceux-ci.

28. Procédé de coloration comprenant l'application, sur une matière kératinique, d'une composition de coloration selon l'une quelconque des revendications 1 à 25, un temps de pose suffisant pour permettre l'obtention de la coloration souhaitée.

29. Procédé de coloration selon la revendication 28, **caractérisé par le fait qu'**il comprend le chauffage des cheveux sur lesquels est appliqué la composition de coloration jusqu'à une température de 250 °C, de préférence de 60 °C.

30. Procédé de coloration comprenant l'application sur la matière kératinique l'une après l'autre, dans n'importe quel ordre, d'une composition (a) et d'une composition (b) telles que définies dans la revendication 26 ou 27.

31. Procédé de coloration selon la revendication 30, qui comprend une étape de rinçage intermédiaire entre l'application de la première composition (a) et l'application de la deuxième composition (b).

32. Procédé de coloration selon l'une quelconque des revendications 28 à 31 pour la coloration des fibres kératiniques.

33. Utilisation d'une composition telle que définie selon l'une quelconque des revendications 1 à 25 pour la teinture des fibres kératiniques.
